Europäisches Patentamt

European Patent Office

Office européen des brevets

(19)

(11) Publication number: **0 053 847**

**A1**

(12) **EUROPEAN PATENT APPLICATION**

(21) Application number: **81201219.3**

(22) Date of filing: **30.10.81**

(51) Int. Cl.³: **C 07 C 29/04**
**C 07 C 35/08, C 07 C 45/00**
**C 07 C 49/403**

(30) Priority: **04.12.80 NL 8006602**

(43) Date of publication of application:
**16.06.82 Bulletin 82/24**

(84) Designated Contracting States:
**AT BE CH DE FR GB IT LI LU NL SE**

(71) Applicant: **STAMICARBON B.V.**
**Postbus 10**
**NL-6160 MC Geleen(NL)**

(72) Inventor: **Boelens, Christiaan**
**Pater Pelzersstraat 48**
**NL-6125 CB Obbicht(NL)**

(72) Inventor: **van Geem, Paul Christiaan**
**Constantijn Hugostraat 13**
**NL-6176 BS Spaubeek(NL)**

(74) Representative: **Pinckaers, August René et al,**
**OCTROOIBUREAU DSM Postbus 9**
**NL-6160 MA Geleen(NL)**

(54) **Process for the preparation of cyclohexanol and/or cyclohexanone.**

(57) The invention relates to a process for the preparation of cyclohexanol and/or cyclohexanone from cyclohexane, in which process the cyclohexane is dehydrogenated to cyclohexene preferably at a temperature of 200 to 700°C and a pressure of 0.01 to 10 Mpa, the cyclohexene hydrated to cyclohexanol preferably at a temperature of −50 to 150°C and a pressure of 0.01 to 10 MPa, and/or oxidized to cyclohexanone preferably at a temperature of 0 to 350°C and a pressure of 0.01 to 200 MPa, the cyclohexanol and/or the cyclohexanone separated, and the unconverted cyclohexane and/or cyclohexene returned to the dehydrogenation step.

EP 0 053 847 A1

STAMICARBON B.V.

0053847

AE 3245

-1-

## PROCESS FOR THE PREPARATION OF CYCLOHEXANOL AND/OR CYCLOHEXANONE

The invention relates to a process for the preparation of cyclohexanol and/or cyclohexanone from cyclohexane. Such a process is known from British patent No. 1,382,849. According to the known process, a mixture of cyclohexanol and cyclohexanone is prepared by oxidizing cyclohexane to cyclohexyl hydroperoxide and then converting this cyclohexyl hydroperoxide with the aid of a metal salt in the presence of an alkali metal hydroxide or carbonate to a mixture of cyclohexanol and cyclohexanone. A disadvantage of the known process is the low degree of conversion to useful products (approx. 75 %). The rest of the starting material is converted to unusable by-products, consisting mainly of a mixture of a large number of mono- and dicarboxylic acids.

According to the invention, a process for the preparation of cyclohexanol and/or cyclohexanone from cyclohexane is characterized in that the cyclohexane is dehydrogenated to cyclohexene, the cyclohexene is hydrated to cyclohexanol and/or oxidized to cyclohexanone, the cyclohexanol and/or the cyclohexanone is separated, and unconverted cyclohexane and/or cyclohexene is returned to the dehydrogenation step. This process has conversion levels to useful products (cyclohexanol, cyclohexanone, benzene) that are much higher than those according to the known process.

In the dehydrogenation of cyclohexane to cyclohexene, benzene is formed as a by-product. This benzene can be separated from the benzene-cyclohexene-cyclohexane mixture remaining after separation of the cyclohexanol and/or the cyclohexanone from the reaction mixture of the hydration and/or oxidation step, to enable the remaining cyclohexane-cyclohexene mixture to be returned to the dehydrogenation step, but benzene is difficult to separate from cyclohexane and/or cyclohexene, so that expensive methods such as extractive distillation will have to be resorted to. According to a very suitable embodiment of the invention, the mixture of benzene and cyclohexane and/or cyclohexene co-obtained in separation of cyclohexanol from the hydration step reaction mixture and/or cyclohexanone from the oxidation step reaction mixture is subjected to a hydrogenation reaction, and the cyclohexane thus obtained returned to the dehydrogenation step.

This process according to the invention has the great advantage that the difficult separation of benzene from mixtures of cyclohexane and/or cyclohexene can be dispensed with, while the conversion with respect to cyclohexanol and/or cyclohexanone rises to approx. 90%. Losses of expensive extractive-distillation agent can be avoided.

The dehydrogenation of cyclohexane to cyclohexene may be effected in any known way. It preferably takes place at a temperature of 200 to 700°C and a pressure of 0.01 to 10 MPa. A suitable method is for instance described in Kinetics and Catalysis, Vol. 20 (2), pp. 323-327 (1979), which for brevity's sake is refferred to. According to the known method, cyclohexene is prepared by oxidative dehydrogenation of cyclohexane by passing cyclohexane at 300-475 °C at atmospheric pressure with air over a zeolitic catalyst. The oxygen-cyclohexane molar ratio should be between 1 : 2 and 3 : 2.

The hydration of cyclohexene to cyclohexanol may be effected in any known way; an acidic catalyst is usually applied. It preferably takes place at a temperature of -50 to 150°C and a pressure of 0.01 to 10 MPa. Very suitable methods are described in British patents 1,381,149 and 1,542,996, which for brevity's sake are referred to. Sulphuric acid is very suitable as catalyst. Iron(II) sulphate may be used as co-catalyst. The hydration is usually effected as a process with separate steps, viz. 1) addition of the acid to the double bond of the cyclohexene, with formation of the ester of cyclohexanol and the acid, e.g. cyclohexyl hydrogen sulphate and 2) hydrolysis of the cyclohexyl ester to cyclohexanol and the acid. The first step is e.g. carried out at a temperature between -50 °C and +30 °C, although the temperatures 30-100 °C are also possible, and the second step at 50-150 °C. The hydration of cyclohexene can also be effected with other catalysts than sulphuric acid, e.g. with a strongly acidic ion exchanger, e.g. a cross-linked polystyrene resin containing sulphonic acid groups, or phosphoric acid. The hydration of the cyclohexene is effected in the presence of the cyclohexane not converted in the dehydrogenation step and the benzene formed therein. The oxidation of cyclohexene to cyclohexanone may be effected in any known way. It preferably takes place at a temperature of 0 to 350°C and a pressure of 0.01 to 200 MPa. A suitable method for the oxidation of cyclohexene is described in Angewandte Chemie, 71 (5), p. 176 et seq. (1959), which for brevity's sake is referred to. An acid aqueous solution of $PdCl_2$ is suitable as catalyst. As co-catalyst, $CuCl_2$, $Fe_2(SO_4)_3$, $K_2Cr_2O_7$, $K_2S_2O_8$ can for instance be applied. The oxidation can be effected as a process with separate steps: in the first step, oxidation of cyclohexene to cyclohexanone is effected by reacting cyclohexene with the catalyst solution, the catalyst being reduced; in the second step, after the organic phase has been separated from the reaction mixture, the catalyst is brought back to the oxidized form with the aid of an oxygen-containing gas (e.g. air) and returned to the first step. The first step can be effected at a temperature between 0 and 150 °C and at a pressure between 0.05 and 5 MPa. The second step can be effected at a temperature between 0 and 250 °C and at a pressure between 0.05 and 200 MPa. The oxidation of the cyclohexene is effected in the presence of the cyclohexane not converted in the dehydrogenation step and benzene formed therein.

After separation of the aqueous phase from the reaction mixture of the hydration and/or oxidation step, an organic phase remains, which, in addition to cyclohexanol and/or cyclohexanone, also contains benzene, cyclohexane and possibly unconverted cyclohexene. Cyclohexanol and/or cyclohexanone are separated from this mixture, e.g. by distillation, a fraction of benzene, cyclohexane and possibly cyclohexene with a boiling point lower than that of cyclohexanol being co-obtained.

In the process according to the invention, there is no need to laboriously separate the latter fraction into its components. On the contrary, it can be added in toto to a hydrogenation step, in which benzene is hydrogenated to cyclohexane. This hydrogenation preferably takes place at a temperature of 100 to 500°C and a pressure of 0.01 to 10 MPa. Any cyclohexene also present is also converted to cyclohexane in this step. The hydrogenation may e.g. be effected in the gas phase, with a catalyst containing an element of Group VIII of the Periodic System of Elements, e.g. ruthenium, palladium, nickel and preferably platinum. Suitable catalysts are e.g. platinum/aluminium oxide, platinum-nickel alloy. The reaction temperature is e.g. 150-400°C, the pressure e.g. 0.01-5 MPa, preferably higher than 0.2 MPa. For a very suitable embodiment, British patent 1,104,275 is for brevity's sake referred to. The hydrogenation may also be effected in the liquid phase, with a catalyst, preferably a suspension catalyst such as Raney Nickel. The reaction temperature is e.g. 150-250 °C, the pressure e.g. 0.3-1 MPa, although higher and lower pressures may be applied but are economically less efficient. For a very suitable embodiment, Petrol. Int., Vol. 14 (10), pp. 14-16 (1974) is for brevity's sake referred to.

The cyclohexane obtained is returned to the dehydrogenation step. Accumulation of undesirable by-products, only a few per cent of which are formed, e.g. methyl cyclopentane, can be avoided by means of a discharge facility at an appropriate position in the process.

Pure cyclohexanol can be obtained by distilation from the raw cyclohexanol obtained. If so desired, the cyclohexanol may be dehydrogenated to cyclohexanone, which can be used as a raw material for the production of caprolactam. Pure cyclohexanone can be obtained, again by distillation, from the raw cyclohexanone obtained in the oxidation.

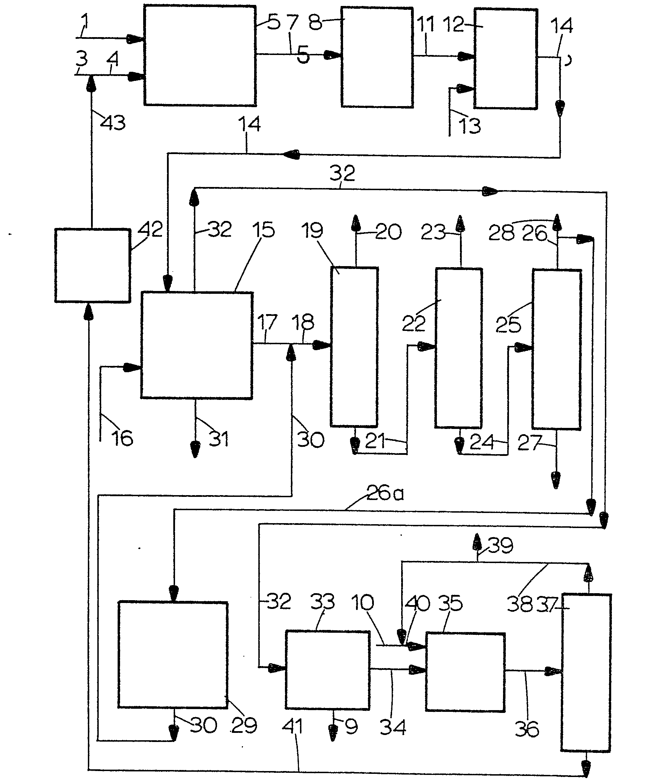

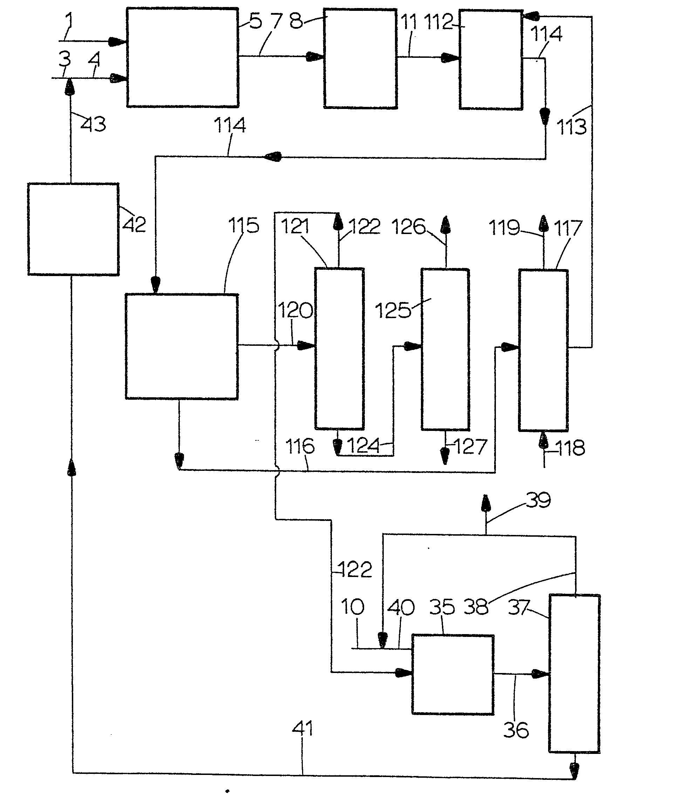

The invention is elucidated further with reference to the accompanying figures. Figure 1 shows a possible flow chart for an embodiment, Figure 2 for another embodiment. The numbers have the same significance in both figures.

Figure 1: Through line 1 air is added, and through line 3/4 cyclohexane, to dehydrogenation reactor 5. Here, the cyclohexane is dehydrogenated with the aid of Na,K erionite at a temperature of 450 °C and atmospheric pressure. The dehydrogenation is effected in the gas phase at an oxygen-cyclohexane molar ratio of 3 : 2. The degree of conversion for cyclohexane is 25 %, while 76 % of the converted cyclohexane is converted to cyclohexene. The by-products consist to a major part of benzene. The gas mixture obtained is fed through line 7 to condensation unit 8. The condensed phase is fed through line 11 to sulphuric acid addition reactor 12. Through line 13, a 60 wt.% aqueous solution of sulphuric acid containing 1 wt.% iron(II) sulphate is added to reactor 12. The addition reaction is effected with reflux cooling, at 80 °C and atmospheric pressure, and at a cyclohexene: sulphuric acid molar ratio of 0.5 : 1. The reaction mixture is subsequently fed to ester hydrolysis reactor 15 through line 14. Through line 16, sufficient water is added to lower the sulphuric acid concentration to 30 wt.%. The ester hydrolysis is effected at 110 °C and atmospheric pressure. The liquid reaction mixture is separated in an undrawn separator into an organic layer consisting of raw cyclohexanol and an aqueous layer containing sulphuric acid, while unconverted cyclohexene, benzene and cyclohexane leave hydrolysis reactor 15 as a vaporous azeotropic mixture with water. Through line 17/18, the organic layer is added together with a cyclohexanone-containing mixture to be described below, to distillation column 19. Here, a light fraction of components with a boiling point lower than that of cyclohexanol and cyclohexanone is distilled off and discharged through line 20. The distillation residue passes through line 21 to a second distillation column 22, where pure cyclohexanone is distilled off and recovered via line 23. The distillation residue is added through line 24 to a third distillation column 25. Here, pure cyclohexanol is distilled off and discharged through line 26. A distillation residue leaves the system through line 27. If so desired, the cyclohexanol may be partly or wholly recovered via line 28, but in this example it passes through line 26a to alcohol dehydrogenation unit 29, where it is (partly) dehydrogenated to cyclohexanone by a known method, e.g. with a zinc or copper catalyst. The hydrogen obtained is separated and returned (not drawn) to hydrogenation reactor 35, and the cyclohexanol/cyclohexanone mixture obtained passes through line 30/18 to distillation column 19. The aqueous layer of the product mixture from ester hydrolysis reactor 15 is discharged through line 31. The sulphuric acid is, if necessary, concentrated e.g. by evaporation and returned to sulphuric acid addition reactor 12. Through line 32, the vaporous mixture of cyclohexene, benzene, cyclohexane and water passes to separator 33. Here, the water is separated from the organic phase and discharged through line 9. The organic phase is fed through line 34 to hydrogenation reactor 35. Hydrogen is fed to reactor 35 through line 10. Here, benzene and cyclohexene are hydrogenated to cyclohexane at 370 °C and 1 MPa with 0.6 wt.% platinum on aluminium oxide as catalyst. Benzene and cyclohexene are converted virtually quantitatively to cyclohexane. The gaseous mixture passes through line 36 to condensor 37. The gas evolved consists mainly of hydrogen, with in addition light by-products such as methyl cyclopentane and methyl cyclopentene. In order to prevent accumulation of these light by-products in the process, part of the gas stream is discharged through line 39. The remainder of the gas stream is returned again to hydrogenation reactor 35 through line 10/40. The condensate, which consists virtually wholly of cyclohexane, is fed through line 41 to evaporator 42. Here, it is evaporated and fed in gaseous form through line 43 to dehydrogenation reactor 5.

Figure 2: The first (dehydrogenation) and the last (hydrogenation) section are the same as described in Figure 1. In the embodiment of Figure 2, the hydration section of Figure 1 is replaced by an oxidation section. For this reason, only this oxidation section is here described.

Now, the organic phase of condensation unit 8 is fed through line 11 to oxidation reactor 112. An aqueous solution containing $PdCl_2$ and $CuCl_2$ is fed to reactor 112 through line 113. The pH of this solution is between -2 and +4. The oxidation reaction is effected at 100 °C at autogenous pressure. The molar ratio of cyclohexene to $PdCl_2$ in the feed to 112 is 100 : 1. The reaction mixture is next fed through line 114 to separator 115, where the catalyst-containing aqueous phase is separated from the organic phase. The aqueous catalyst solution is fed through line 116 to reoxidation reactor 117. Air is fed to reactor 117 through line 118. The reoxidation is effected at 100 °C and 1 MPa. The reoxidized catalyst solution is returned through line 113 to oxidation reactor 112. The exhausted air is discharged through 119. The organic phase from separator 115 is fed through line 120 to distillation unit 121. Here, a mixture of benzene, cyclohexene and cyclohexane is distilled off. The bottom product of distillation unit 121 is fed through line 124 to a second distillation column 125. Here, pure cyclohexanone is distilled off and recovered via line 126. The distillation residue from 125 leaves the system through 127. The benzene-cyclohexene-cyclohexane mixture from 121 is added to hydrogenation reactor 35 through line 122.

From here onwards, the description of Figure 2 is the same as the description of Figure 1 (see above).

C L A I M S

1. Process for the preparation of cyclohexanol and/or cyclohexanone from cyclohexane, characterized in that the cyclohexane is dehydrogenated to cyclohexene preferably at a temperature of 200 to 700 °C and a pressure of 0.01 to 10 MPa, the cyclohexene hydrated to cyclohexanol preferably at a temperature of -50 to 150°C and a pressure of 0.01 to 10 MPa, and/or oxidized to cyclohexanone preferably at a temperature of 0 to 350 °C and a pressure of 0.01 to 200 MPa, the cyclohexanol and/or the cyclohexanone separated, and the unconverted cyclohexane and/or cyclohexene returned to the dehydrogenation step.

2. Process according to Claim 1, characterized in that the mixture of benzene and cyclohexane and/or cyclohexene co-obtained in separation of cyclohexanol from the hydration step reaction mixture and/or cyclohexane from the oxidation step reaction mixture is subjected to a hydrogenation reaction preferably at a temperature of 100 to 500 °C and a pressure of 0.01 to 10 MPa, and the cyclohexane thus obtained returned to the dehydrogenation step.

3. Process according to Claim 1 or 2, characterized in that the hydrogenation reaction is effected in the gas phase at a temperature between 150 and 400 °C and a pressure between 0.01 and 5 MPa.

4. Process according to Claim 3, characterized in that a pressure of above 0.2 MPa is applied.

5. Process according to Claim 3 or 4, characterized in that platinum is used as catalyst in the hydrogenation reaction.

6. Process according to Claim 1 or 2, characterized in that the hydrogenation reaction is effected in the liquid phase at a temperature between 150 and 250 °C.

7. Process according to Claim 6, characterized in that a pressure between 0.3 and 1 MPa is applied.

8. Process according to Claim 1, as is substantially described in the specification and/or the figures.

9. Cyclohexanol and/or cyclohexanone obtained by means of the process according to any of the foregoing claims.

2/2

0053847

0053847

## DOCUMENTS CONSIDERED TO BE RELEVANT

| Category | Citation of document with indication, where appropriate, of relevant passages | Relevant to claim |
|---|---|---|
| E | EP - A - 0 023 379 (STAMICARBON)<br>* Whole document * | 1-9 |
| A | DE - A - 1 087 595 (RUHRCHEMIE AG)<br>* Column 1,2 * | |
| Y | FR - A - 2 280 616 (BAYER)<br>* Page 1, line 36 - page 2, line 32; page 3, line 30 - page 4, line 19 * | 2-7 |
| Y | EP - A - 0 005 293 (STAMICARBON)<br>* Page 2, lines 9-27 * | 2-7 |
| A | GB - A - 939 613 (SCIENTIFIC DESIGN CY.)<br>* Page 1, lines 13-51 * | |
| Y | CHEMICAL ABSTRACTS, vol. 86, no. 17, 25th April 1977, page 508. no. 120882m<br>Columbus, Ohio, U.S.A.<br>& JP - A - 76 118 743 (UBE INDUSTRIES, LTD.) 18-10-1976<br>* Abstract * | 2 |
| Y | FR - A - 2 442 835 (INSTITUT FRANCAIS DU PETROLE)<br>* Page 3, line 24 - page 4, line 7; page 7 *<br>-- ./. | 1 |

CLASSIFICATION OF THE APPLICATION (Int. Cl. 3)

C 07 C 29/04
　　　　35/08
　　　　45/00
　　　　49/403

TECHNICAL FIELDS SEARCHED (Int.Cl. 3)

C 07 C 45/34
　　　　49/303
　　　　29/03
　　　　29/04
　　　　29/06

The present search report has been drawn up for all claims

| Place of search | Date of completion of the search | Examiner |
|---|---|---|
| The Hague | 06-01-1982 | SCHRYVERS |

EPO Form 1503.1 06.78

European Patent
Office

**EUROPEAN SEARCH REPORT**

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| Y | PATENTS ABSTRACTS OF JAPAN, vol. 2, no. 126, 21st October 1978, abstract no. 2527C78<br>& JP - A - 53 90242, 8th August 1978 (TORAY K.K.)<br>* Abstract * | 1 | |
| | ----- | | |
| | | | TECHNICAL FIELDS SEARCHED (Int. Cl.³) |

EP 81 20 1219

EPO Form 1503.2  06.78